# EUROPEAN PATENT APPLICATION

(11) **EP 1 266 642 A1**
(43) Date of publication of application: **18.12.2002**
(21) Application number: 01114373.2
(22) Date of filing: 13.06.2001
(51) Int. Cl.: A61F 2/44

(54) **Intervertebral retrieval device**

(71) Applicant: Lin, Chih-I, Chino Hills, CA 91709 (US)
(72) Inventor: Lin, Chih-I, Chino Hills, CA 91709 (US)
(74) Representative: Grünecker, Kinkeldey, Stockmair & Schwanhäusser Anwaltssozietät

(57) **Abstract**

An intervertebral retrieval device comprises a block body (100) and a bone nail (210,220,230). The block body is provided in an upper surface (120) thereof and a lower surface (170) thereof with a plurality of protruded teeth (110)(160), with at least one of the two contact surfaces having an inclined hole (130). The bone nail (210,220,230) is fastened onto a vertebra from the upper side or the lower side of a replacing intervertebral disc such that the bone nail (210,220,230) penetrates slantingly the vertebra or its replacing body or similar device, and that the bone nail is inserted into the inclined hole (130) of the block body (100).

## Description

The present invention provides an intervertebral retrieval device comprising a block body provided on an upper surface and a lower surface thereof with a plurality of protruded teeth, and with an inclined hole at at least one of said upper surface and lower surface; and
a bone nail fastened onto a vertebra contacting the upper surface or the lower surface of said block body with a portion of the bone nail inserting into said inclined hole of said block body.

In spite of the use of the device of the present invention in conjunction with the auxiliary fixation device, the intervertebral retrieval device of the present invention allows a relatively greater freedom of intervertebral movement between the vertebrae. This is due to the fact that the bone nail and the inclined hole of the block body of the present invention are not joined together intimately. In light of the bone nail being fastened onto the vertebra, the block body and the vertebrae are fused together.

### BRIEF DESCRIPTION OF THE DRAWINGS

Fig. 1 shows a perspective view of a block body of the intervertebral retrieval device of the present invention.
Fig. 2 shows a schematic partial cross sectional view of the present invention comprising two block bodies, three bone nails, and the auxiliary fixation device.
Fig. 3 shows a schematic view of the intervertebral retrieval devices of the present invention as shown in FIG. 2 implanted in a spine.
Fig. 4 shows a schematic view of the intervertebral retrieval device of the present invention being used to replace a single deformed intervertebral disc.

### DETAILED DESCRIPTION OF THE EMBODIMENTS

The intervertebral retrieval device of the present invention comprises a block body and a bone nail.

The block body is provided in the upper surface thereof and the lower surface thereof with a plurality of protruded teeth, and with an inclined hole at at least one of the upper and lower surfaces.

The bone nail is fastened onto an upper vertebra or a lower vertebra contacting said block body, such that the bone nail penetrates slantingly the upper vertebra or the lower vertebra, and that the bone nail is then received in the inclined hole of the block body. The upper vertebra or the lower vertebra onto which the bone nail is fastened can also be a replacing body or similar device of a vertebra.

The block body may be of any shape. The upper surface and the lower surface of the block body are a convex surface. In this specification, the "convex surface" of the block body means the surface protruded outwardly and in an arcuate manner at the portion between the ends of the block body. In this specification, the words, such as upper, lower, front, rear, left, right, refer to the upper side, the lower side, the front side, the rear side, the left side, and the right side of a human body in which the block body is implanted. The protruded teeth of the present invention can be any kinds protruded teeth known in the art, for examples random or regular cones, and parallel lateral teeth, and preferably parallel lateral teeth. The inclined hole means inclination from the upright position. Preferably, said upper surface and said lower surface of said block body each has an inclined hole.

The bone nail of the present invention is the prior art bone nail.

The bone nail may be additionally fastened onto an auxiliary vertebral fixation device.

The bone nail may be fixedly received (such as threaded) in the inclined hole of the block body. The bone nail may be inserted into the inclined hole of the block body such that the bone nail is not substantially connected with the inner wall of the inclined hole of the block body.

It is preferable that the bone nail is not fixedly received in the inclined hole of the block body, so as to allow a greater micromotion between the block body and the bone nail (the vertebrae), thereby promoting the bone ingrowth. In light of the bone nail being inserted slantingly into the inclined hole of the block body, the block body can be pulled out in the direction consistent with the inclination of the inclined hole of the block body. However, the block body is not allowed to move in this direction by the vertebrae. In other words, the block body will not slip out the bone nail, even though the bone nail and the inclined hole of the block body are not substantially joined together.

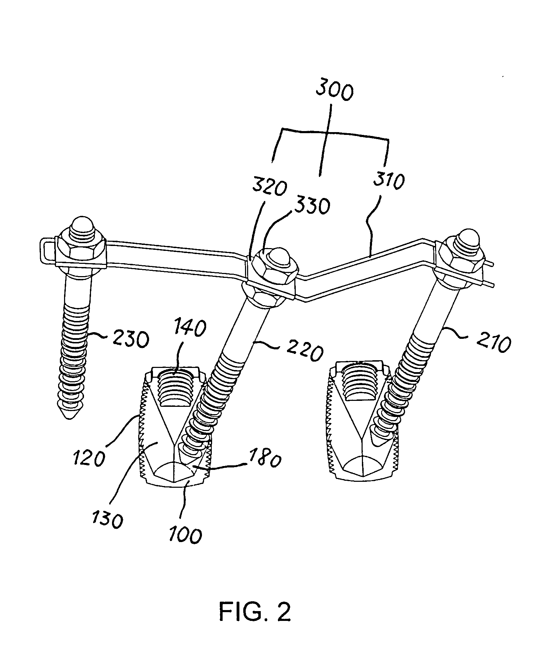

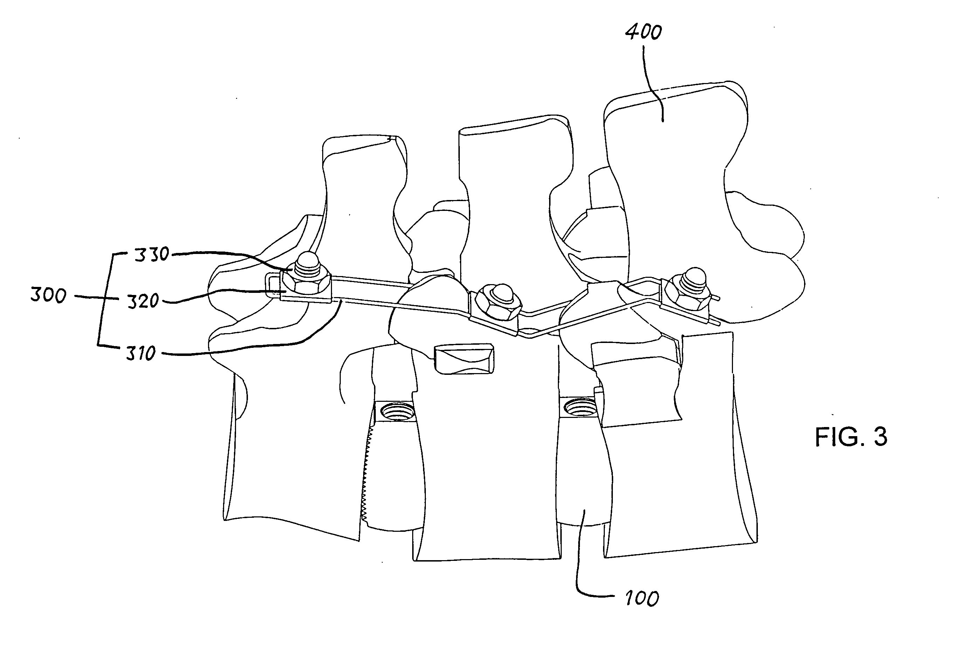

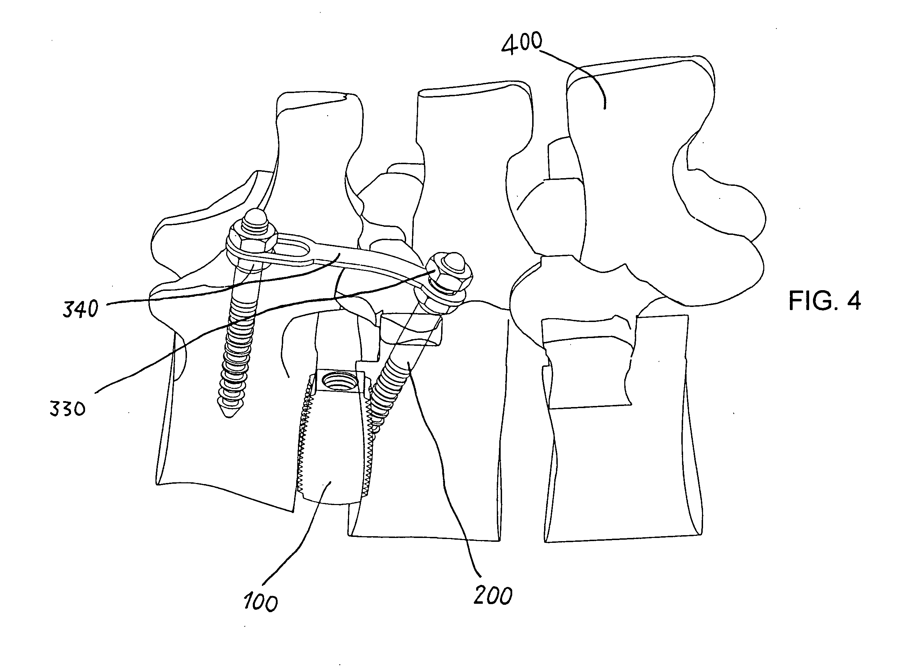

The intervertebral retrieval device of the present invention may be assisted by the auxiliary fixation device of the prior art, such as the auxiliary fixation device formed of an auxiliary fixation plate and auxiliary fixation screw nuts, as shown in FIG. 4, or the auxiliary fixation device formed of an auxiliary fixation thread/auxiliary fixation pieces/auxiliary fixation screw nuts, as shown in FIGS. 2 and 3.

The block body, the bone nail and the auxiliary device of the present invention are made of biomaterials compatible biologically with the orthopedic surgery, such as stainless 316LVM, Ti-6-4, the cobalt-molybdenum-nickel alloy, etc.

The aforementioned block body comprises at least one inclined hole, preferably two inclined holes symmetrical and opposite to each other. The inclined hole is located anywhere in the upper or lower surface of the block body such that the inclined hole extends inwardly. The inclined hole is preferably located at the center of the upper or lower surface of the block body.

The present invention will be more readily understood upon a thoughtful deliberation of the following description of a preferred embodiment with reference to the accompanying drawings.

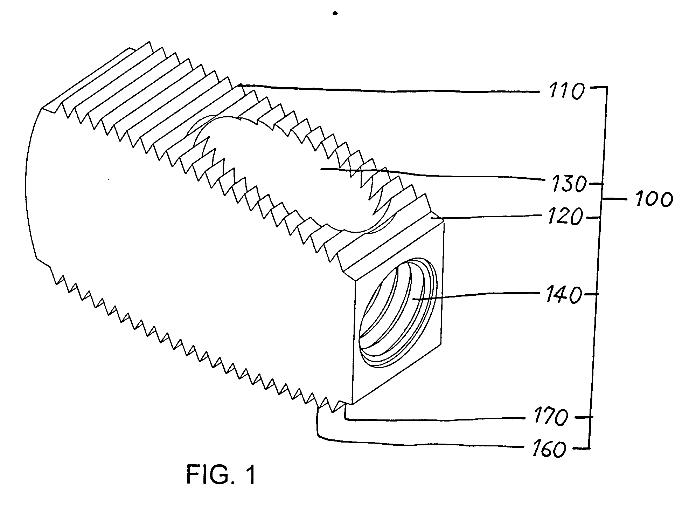

In FIG. 1, the block body 100 has the upper surface 120, the protruded teeth 110, and the inclined hole 130, the protruded teeth 160, the lower surface 170, and the tool hole 140. The protruded teeth 110 and 160 are formed of stripe-shaped teeth parallel to one another. The inclined hole 130 is devoid of threads.

In FIG. 2, the reference numerals of 100, 110, 120, 130, 140, 160, 170 are similar in definition to those in FIG. 1. The lower surface of the block body 100 has an inclined hole 180. Tails of bone nails 210, 220, and 230 to be fixed onto vertebrae are received in the inclined holes of the block bodies after being fastened to an auxiliary fixation device comprising a fixation thread 310, and three fixation pieces 320, and three fixation screw nuts 330.

## Claims

1. An intervertebral retrieval device comprising
a block body provided on an upper surface and a lower surface thereof with a plurality of protruded teeth, and with an inclined hole at at least one of said upper surface and lower surface; and
a bone nail to be fastened onto a vertebra contacting the upper surface or the lower surface of said block body with a portion of the bone nail inserting into said inclined hole of said block body.

2. The intervertebral retrieval device as defined in claim 1, wherein said upper surface and said lower surface are a convex surface.

3. The intervertebral retrieval device as defined in claim 2, wherein said protruded teeth of said block body are parallel lateral teeth.

4. The intervertebral retrieval device as defined in claim 1, wherein said upper surface and said lower surface of said block body each has an inclined hole.

5. An intervertebral retrieval device comprising:
a plurality of block bodies, each of which is provided on an upper surface and a lower surface thereof with a plurality of protruded teeth, and with an inclined hole at at least one of said upper surface and lower surface;
a plurality of bone nails, each of which is to be fastened onto a vertebra contacting the upper surface or the lower surface of said block body with a portion of the bone nail inserting into said inclined hole of said block body; and
an auxiliary fixation device enabling said bone nails to join together.

6. The intervertebral retrieval device as defined in claim 5, wherein said upper surface and said lower surface are a convex surface.

7. The intervertebral retrieval device as defined in claim 6, wherein said protruded teeth of said block body are parallel lateral teeth.

8. The intervertebral retrieval device as defined in claim 5, wherein said upper surface and said lower surface of said block body each has an inclined hole.
